# EUROPEAN PATENT APPLICATION

(11) **EP 4 737 583 A1**
(43) Date of publication of application: **06.05.2026**
(21) Application number: 24832048.3
(22) Date of filing: 27.06.2024
(51) Int. Cl.: C12P 21/02, C07K 14/47, C07K 16/00, C12N 1/21, C12N 9/90, C12N 15/12, C12N 15/13, C12N 15/61, C12P 21/08

(54) **METHOD FOR PRODUCING PROTEIN OF INTEREST**

(30) Priority: 28.06.2023 JP 2023105948; 22.02.2024 JP 2024025618
(71) Applicant: Kao Corporation, Tokyo 103-8210 (JP)
(72) Inventor: NISHIGUCHI, Hiroki, Tokyo 103-8210 (JP); KAWAHARA, Akihito, Tokyo 103-8210 (JP)
(74) Representative: Vossius & Partner Patentanwälte Rechtsanwälte mbB
(86) International application number: PCT/JP2024/023366
(87) International publication number: WO 2025/005189

(57) **Abstract**

Provided is a method for producing a protein of interest using a gram-positive bacterium with improved productivity. The method for producing a protein of interest comprises: culturing a gram-positive bacterium comprising a gene encoding heterologous FK506-binding protein (FKBP) and a gene encoding the protein of interest; or culturing a mixture of a gram-positive bacterium comprising the gene encoding heterologous FKBP and a gram-positive bacterium comprising the gene encoding the protein of interest.

## Description

### Field of the Invention

The present invention relates to a method for producing a protein of interest with improved productivity.

### Background of the Invention

Various types of useful substances are produced industrially using microbes, including foods, amino acids, organic acids, nucleic acid-related substances, antibiotics, carbohydrates, lipids, proteins, and the like, and their application falls within a wide range of fields from daily goods such as foods, medicines, detergents, and cosmetics to various starting materials for chemical products. Examples of industrially useful host microbes include *E. coli, Bacillus subtilis,* yeasts, and filamentous fungi. An important challenge to the industrial production of useful substances using such microbes is improvement in its productivity.

Peptidylprolyl isomerase (PPIase) is an enzyme capable of catalyzing the isomerization of prolyl bonds and assisting in protein folding (Non Patent Literature 1). PPIase consists of three families: cyclophilin, FK506-binding protein (FKBP), and parvulin, differing in sensitivity to inhibitors. The amino acid sequences of these three families of PPIase are known to share low sequence identity. All the three families of PPIase reside in the periplasm of *E. coli.* Among them, FkpA which belongs to FKBP has been studied in detail and reported to be capable of improving the productivity of a protein of interest with increase in its expression level (Non Patent Literature 2). It has also been reported that the region of FkpA which contributes to improvement in the productivity of a protein of interest in *E. coli* is an N-terminal chaperon region (Non Patent Literature 3). Moreover, the addition of PPIase belonging to any of the three families has been reported to be capable of improving the productivity of a protein of interest in a cell-free system (Patent Literature 1). Enhanced expression of parvulin including PrsA has often been studied on the secretory production of a protein of interest in gram-positive bacteria (Non Patent Literature 4).

It has been reported that a gram-positive bacterium *Bacillus subtilis* has PrsA as endogenous chaperon and PPIase (Non Patent Literature 5), and the productivity of a protein of interest is improved by enhancing the expression level of PrsA (Patent Literature 2 and Non Patent Literatures 6 and 7). PrsA is anchored to the outer surface of a cell membrane and promotes the folding of a protein transported via translocase. Since incorrectly folded proteins are degraded by protease present in cell walls or cell membrane-cell membrane interfaces, correct folding is required immediately after cleavage of a signal peptide (Non Patent Literature 4).

For FKBP, however, there has been no report so far regarding its influence on the production of a protein of interest in gram-positive bacteria.

(Patent Literature 1) JP-A-2005-253432
(Patent Literature 2) JP-B-4202985

(Non Patent Literature 1) Journal of Molecular Biology, 2015, 427 (7): 1609-1631
(Non Patent Literature 2) Biotechnol Prog. 2017, 33 (1): 212-220
(Non Patent Literature 3) Microbial Cell Factories, 2010, volume 9, Article number: 22
(Non Patent Literature 4) Microbial Cell Factories, 2019, volume 18, Article number: 158
(Non Patent Literature 5) J Biol Chem. 2004, 279 (18): 19302-14
(Non Patent Literature 6) Journal of Bacteriology, 1998, 180 (11): 2830-2835
(Non Patent Literature 7) Journal of Bacteriology, 2001, 183 (6): 1881-1890

### Summary of the Invention

The present invention relates to the following 1) and 2).
1) A method for producing a protein of interest, comprising: culturing a gram-positive bacterium comprising a gene encoding heterologous FKBP and a gene encoding the protein of interest; or culturing a mixture of a gram-positive bacterium comprising the gene encoding heterologous FKBP and a gram-positive bacterium comprising the gene encoding the protein of interest.
2) A gram-positive bacterium comprising a gene encoding heterologous FKBP, or the gene encoding heterologous FKBP and a gene encoding a protein of interest.

### Brief Description of the Drawings

Fig. 1 illustrates an amount of Fab produced in a Fab-producing *Bacillus subtilis* strain allowed to express each PPIase.
Fig. 2 illustrates an amount of Fab produced in a Fab-producing *Bacillus subtilis* strain allowed to express PrsA or FkpA.
Fig. 3 illustrates an amount of Fab produced in a Fab-producing *Bacillus subtilis* strain allowed to express the whole region (FkpA), an N-terminal region (FkpA_N) or a C-terminal region (FkpA_C) of FkpA.
Fig. 4 illustrates an amount of Fab produced in a Fab-producing *Bacillus subtilis* strain allowed to secretorily express FkpA (FkpA_p) or allowed to express FkpA anchored to a cell membrane (FkpA_m).
Fig. 5 illustrates the phylogenetic tree of FKBP.
Fig. 6 illustrates the HMM profile of BCFK clade.

### Detailed Description of the Invention

As used herein, the identity between nucleotide sequences or amino acid sequences is calculated by the Lipman-Pearson method (Science, 1985, 227: 1435-1441). Specifically, the identity is calculated by analysis using the homology analysis (search homology) program of genetic information processing software Genetyx-Win in which the unit size to compare (ktup) is set to 2.

As used herein, "at least 80% identity" regarding an amino acid sequence or a nucleotide sequence refers to 80% or higher, preferably 85% or higher, more preferably 90% or higher, further more preferably 95% or higher, further more preferably 96% or higher, further more preferably 97% or higher, further more preferably 98% or higher, further more preferably 99% or higher identity.

As used herein, "corresponding position" or "corresponding region" on an amino acid sequence or a nucleotide sequence can be determined by aligning a sequence of interest and a reference sequence (e.g., an amino acid sequence of SEQ ID NO: 1) so as to give the largest homology. The alignment of amino acid sequences or nucleotide sequences can be carried out using an algorithm known in the art, and its procedures are known to those skilled in the art. The alignment can be performed by using, for example, Clustal W multiple alignment program (Nucleic Acids Res, 1994, 22: 4673-4680) at default settings. Alternatively, Clustal W2 or Clustal omega, a modified version of Clustal W, may be used. Clustal W, Clustal W2, and Clustal omega are available on, for example, the website of Clustal run by University College Dublin ([www.clustal.org]) or the website of European Bioinformatics Institute (EBI) [www.ebi.ac.uk/index.html]) or DNA Data Bank of Japan (DDBJ) run by National Institute of Genetics [www.ddbj.nig.ac.jp/searches-j.html]). The position of the sequence of interest aligned to an arbitrary position of the reference sequence by the alignment mentioned above is regarded as "position corresponding" to the arbitrary position. A region flanked by corresponding positions or a region consisting of a corresponding motif is regarded as a corresponding region.

Those skilled in the art can further optimize the alignment of amino acid sequences thus obtained by fine adjustment. Such optimum alignment is preferably determined in consideration of the identity or similarity of amino acid sequences, the frequency of an inserted gap, and the like. In this context, the identity or similarity of amino acid sequences refers to, when two amino acid sequences are aligned, the ratio (%) of the number of positions at which the same or similar amino acid residues reside in both the sequences to the number of full-length amino acid residues. The similar amino acid residues mean amino acid residues which have properties similar to each other in terms of polarity or charge among 20 amino acids constituting a protein, and which cause so-called conservative substitution. Groups of such similar amino acid residues are well known to those skilled in the art. Examples thereof include, but are not limited to: arginine and lysine; glutamic acid and aspartic acid; serine and threonine; glutamine and asparagine; and leucine and isoleucine.

As used herein, "amino acid residue" means any of 20 amino acid residues constituting a protein, i.e., alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

As used herein, "operable linking" of a gene to a control region means that the gene is linked to the control region such that the gene is expressible under the control of the control region. Procedures of "operable linking" of the gene to the control region are well known to those skilled in the art.

As used herein, "upstream" and "downstream" regarding a gene refers to "upstream" and "downstream" in the direction of transcription of the gene. For example, "gene located downstream of a promoter" means that the gene resides on the 3' side of the promoter in a DNA sense strand. "Upstream of a gene" means a region on the 5' side of the gene in a DNA sense strand.

The name of a gene of *Bacillus subtilis* described in the present specification is described on the basis of *Bacillus subtilis* genome data published on the Internet ([bacillus.genome.ad.jp/], updated on January 18, 2006) by JAFAN: Japan Functional Analysis Network for *Bacillus subtilis* (BSORF DB). The gene position of *Bacillus subtilis* described in the present specification refers to a gene position registered in BSORF DB.

As used herein, the FK506-binding protein (FKBP) refers to an enzyme constituting one family of peptidylprolyl isomerase (PPIase, EC5.1.2.8) capable of catalyzing the isomerization of prolyl bonds and assisting in protein folding. FKBP is a group of enzymes whose activity is inhibited by binding to an immunosuppressant FK506 among the PPIase families.

As used herein, "clade" is a population constituted by sequences in a common ancestor and all descendants derived from the common ancestor (evolution.berkeley.edu/evolibrary/article/0_0_0/evo_06). The clade can be visualized as a phylogenetic tree and shares common characteristics. A subclade which forms a group in the clade of the phylogenetic tree can also share common characteristics, and sequences in a certain subclade are more closely related to each other than to sequences in other subclades in the clade.

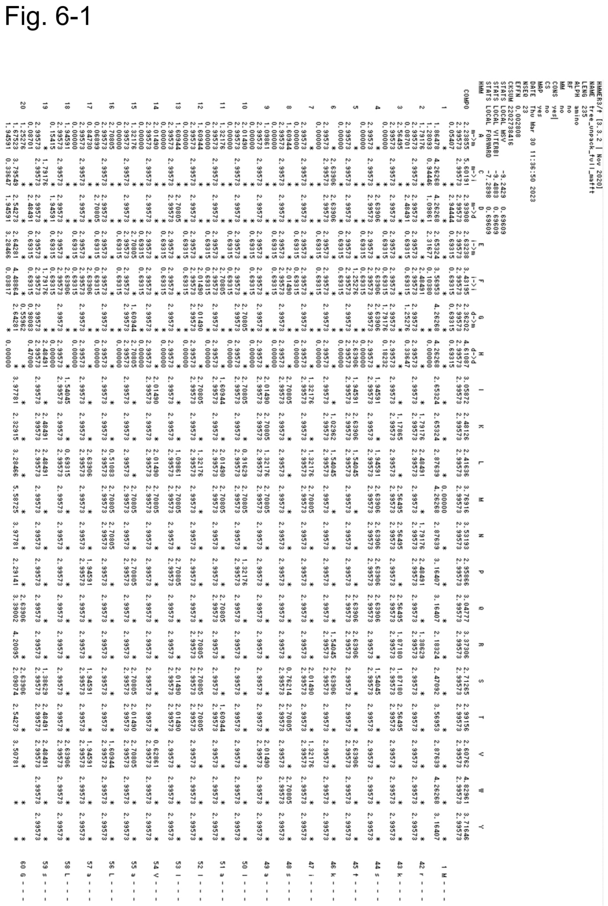

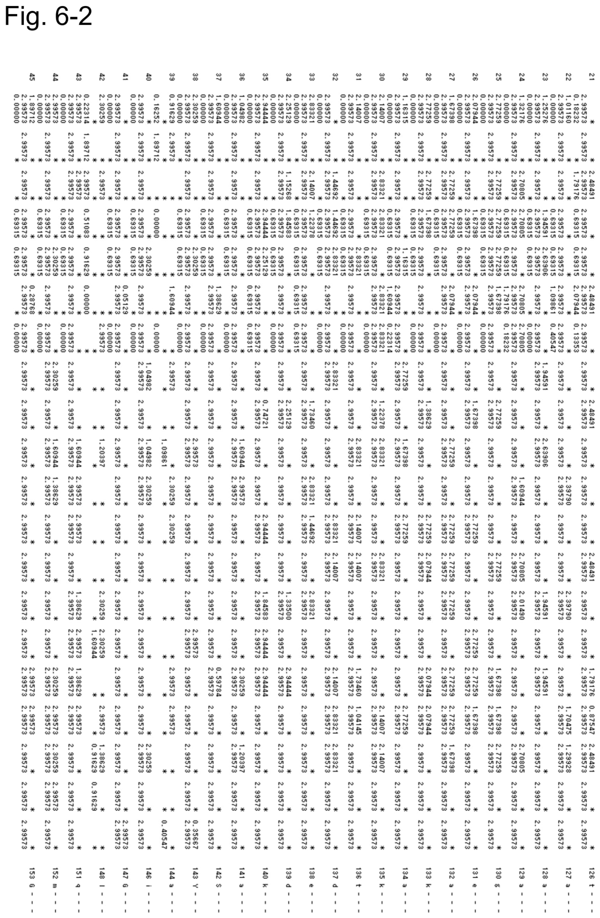

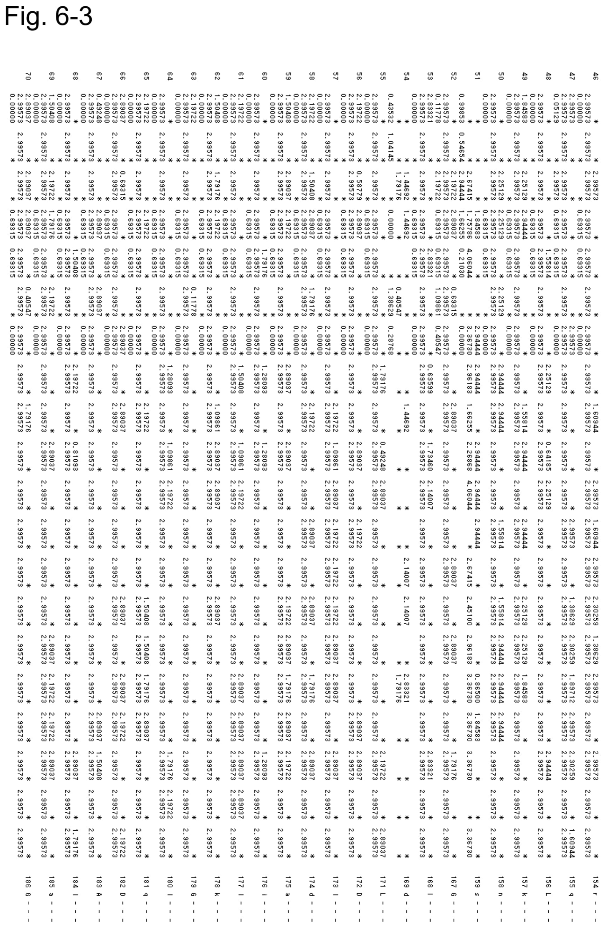

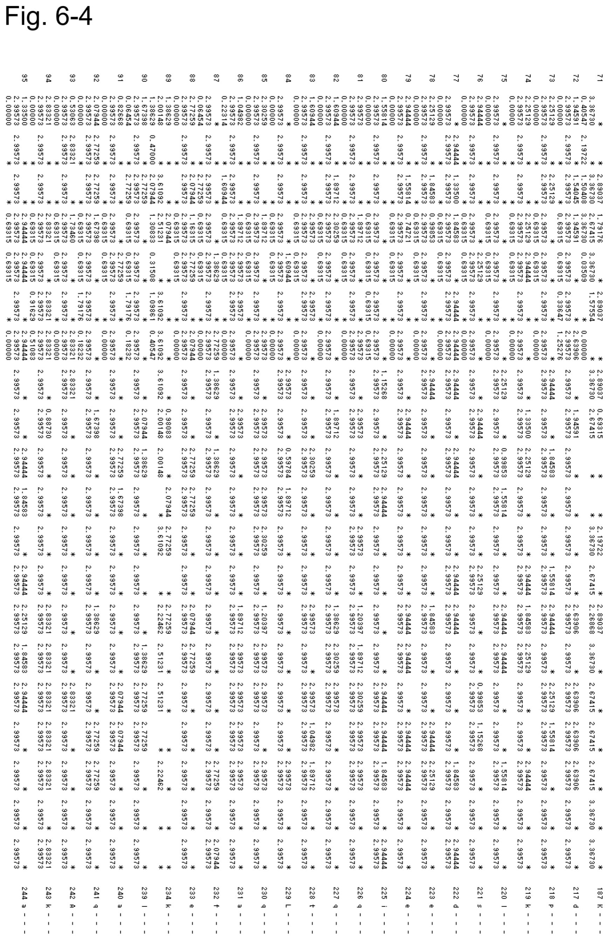

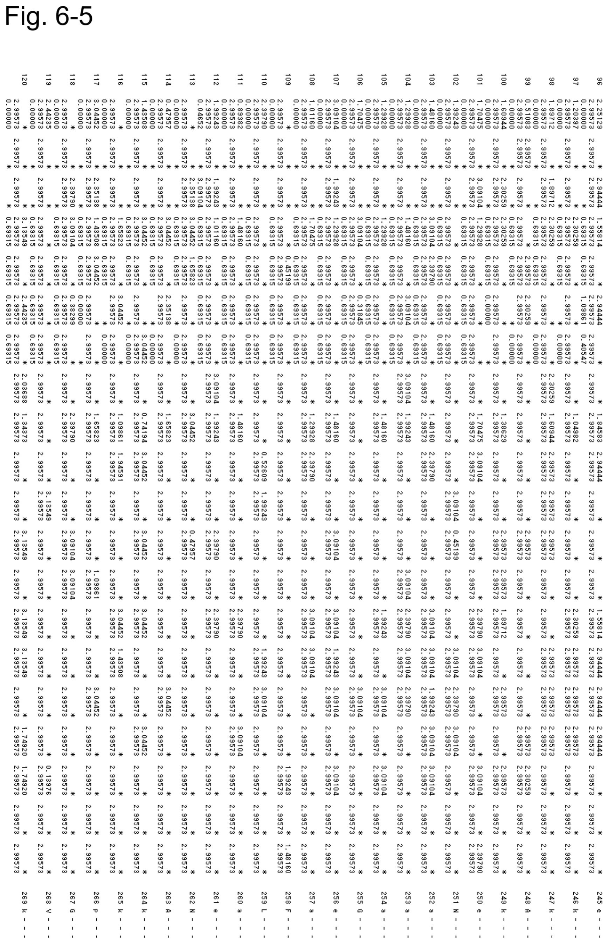

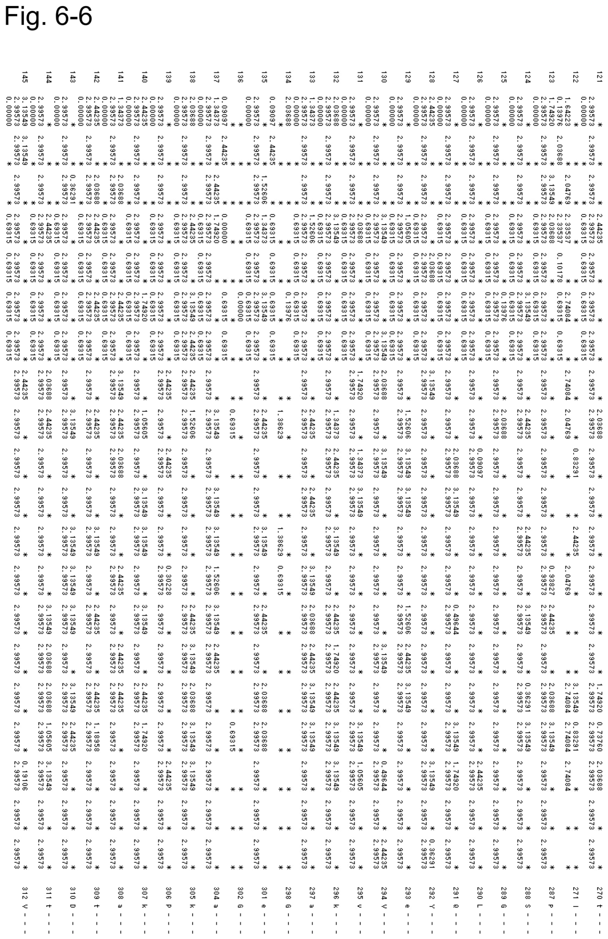

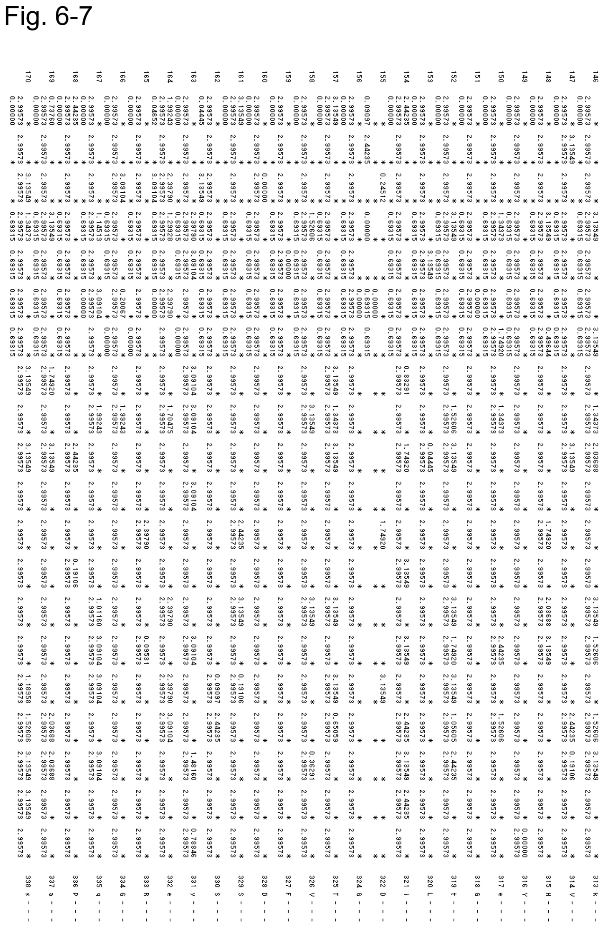

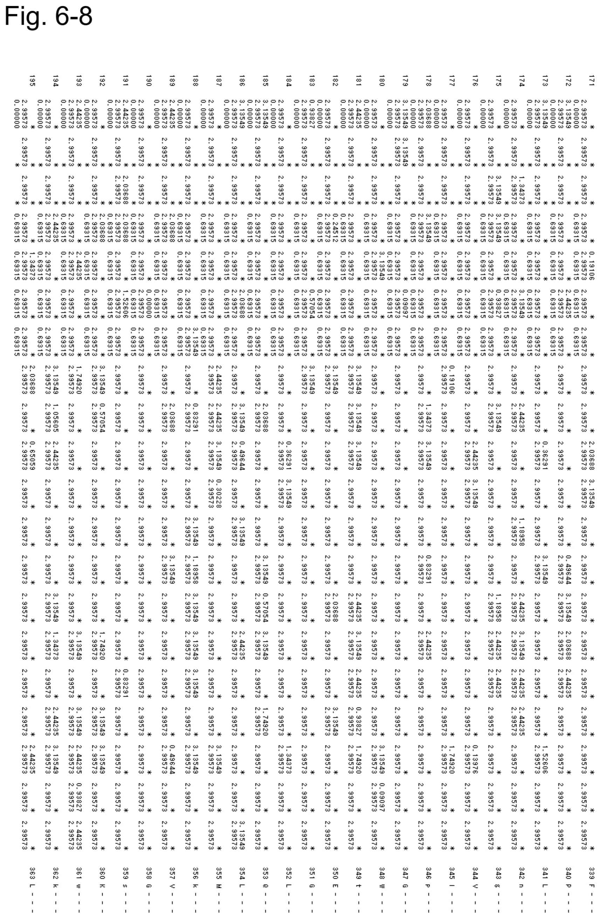

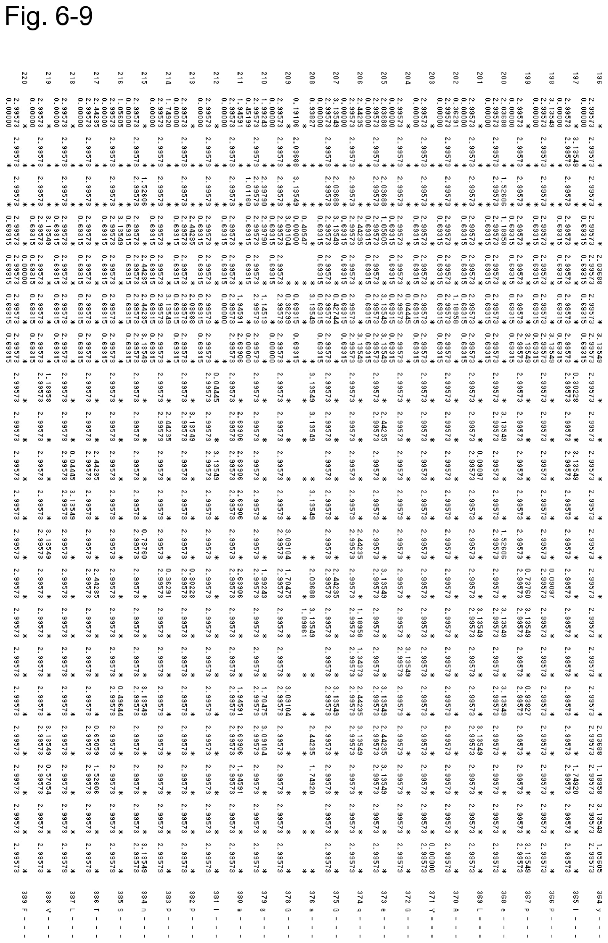

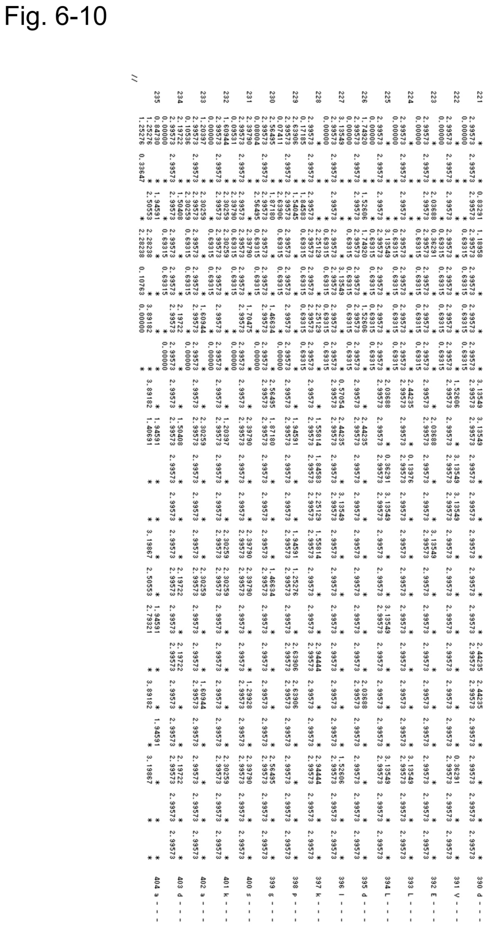

As used herein, "BCFK clade" is a group of sequences which belong to the FKBP family. The sequences which belong to the BCFK clade are defined as sequences having a hidden Markov model (HMM) score of 115 or more in the full-length amino acid sequence based on an HMM profile described in Fig. 6. A preferred sequence which belongs to the BCFK clade is a sequence having an HMM score of 150 or more in the full-length amino acid sequence based on the HMM profile described in Fig. 6.

The HMM profile of Fig. 6 was prepared by constructing multiple alignment with MAFFT v7.471 using an FKBP sequence group belonging to the BCFK clade, and then designating --pnone --wnone as a parameter using HMMER version 3.3.2 (http://hmmer.org/). The uniprot numbers of the FKBP sequence group used in the preparation of the HMM profile which defines the BCFK clade are shown in Table 3 mentioned later. The HMM score of an arbitrary sequence based on the prepared HMM profile of the BCFK clade can be calculated by binarizing the HMM profile using hmmpress, followed by homology search using hmmscan.

As used herein, "antibody-related molecule" refers to an immunoglobulin, or a protein containing a molecular species composed of a single domain or a combination of two or more domains selected from domains constituting the immunoglobulin. Examples of the domains constituting the immunoglobulin include immunoglobulin heavy chain domains VH, CH1, CH2, and CH3, and immunoglobulin light chain domains VL and CL. The antibody-related molecule may be a monomeric protein or may be a multimeric protein. The antibody-related molecule, when being a multimeric protein, may be a homomultimer composed of identical subunits or may be a heteromultimer composed of two or more types of subunits. Preferably, the antibody-related molecule is a molecule which contains an antigen recognition domain of an immunoglobulin and is capable of specifically binding to a target such as a protein. Examples of the antibody-related molecule include immunoglobulins such as IgG, and small-molecule antibodies such as a Fab, a F(ab')₂, a single chain antibody (scFv), a diabody, and a variable domain of the heavy chain of a heavy chain antibody (VHH).

As used herein, a "Fab" refers to an antibody composed of a VH-CH1 domain of an immunoglobulin heavy chain and a VL-CL domain of an immunoglobulin light chain.

As used herein, a "VHH" refers to a variable domain of the heavy chain of a heavy chain antibody. The heavy chain antibody is found in animals of the family *Camelidae,* cartilage fishes such as shark, and the like. The heavy chain antibody derived from cartilage fishes such as shark is also called VNAR (single variable new antigen receptor domain antibody). In the present specification, the VHH is derived from, for example, an animal of the family *Camelidae* or shark, preferably derived from the animal of the family *Camelidae.* The VHH is a molecule which can recognize an antigen by a single domain and is the smallest unit among antibody molecules found to date. The VHH to be produced in the present invention can contain one or more heavy chain variable domains derived from the heavy chain antibody and the number of heavy chain variable domains contained in the VHH is not limited. The heavy chain variable domain contained in the VHH to be produced in the present invention may be a heavy chain variable domain derived from a natural heavy chain antibody or may be a variant thereof. The VHH to be produced in the present invention may contain a fragment other than the heavy chain variable domain derived from the immunoglobulin heavy chain (e.g., a fragment of a constant region of the heavy chain antibody, a human-derived fragment, or a linker sequence), an amino acid mutation for stabilization, and the like.

As used herein, examples of the animal of the family *Camelidae* include Bactrian camel, Arabian camel, llama, alpaca, vicugna, and guanaco and preferably include alpaca.

The present invention relates to providing a method for producing a protein of interest using a gram-positive bacterium typified by *Bacillus subtilis* with improved productivity.

The present inventors conducted diligent studies in light of the object described above and consequently found that in the production of a protein of interest using a gram-positive bacterium, the productivity of the protein of interest can be improved by allowing the gram-positive bacterium to express specific PPIase derived from a heterologous organism.

The present invention enables a protein of interest to be efficiently produced using a gram-positive bacterium typified by *Bacillus subtilis.*

The method for producing a protein of interest according to the present invention contains: culturing a gram-positive bacterium containing a gene encoding heterologous FKBP and a gene encoding the protein of interest; or culturing a mixture of a gram-positive bacterium containing the gene encoding heterologous FKBP and a gram-positive bacterium containing the gene encoding the protein of interest.

In the method of the present invention, FKBP is not particularly limited as long as it is heterologous FKBP. In this context, the term "heterologous" means a microbe or an organism classified into a species other than the species to which the gram-positive bacterium for producing the protein of interest belongs. The heterologous FKBP is preferably a polypeptide which belongs to a BCFK clade and has an isomerization activity of a prolyl bond, a polypeptide consisting of an amino acid sequence of SEQ ID NO: 6, or a polypeptide which consists of an amino acid sequence having at least 80% identity to the amino acid sequence of SEQ ID NO: 6 and has an isomerization activity of a prolyl bond, more preferably a polypeptide consisting of any of the amino acid sequences of SEQ ID NOs: 1 to 6, or a polypeptide which consists of an amino acid sequence having at least 80% identity to any of the amino acid sequences of SEQ ID NOs: 1 to 6 and has an isomerization activity of a prolyl bond, from the viewpoint of improvement in the productivity of the protein of interest.

The polypeptide consisting of the amino acid sequence of SEQ ID NO: 6 is MJFKS derived from *Methanococcus jannaschii.* The polypeptide consisting of the amino acid sequence of SEQ ID NO: 1 is FkpA derived from *E. coli.* The polypeptide consisting of the amino acid sequence of SEQ ID NO: 2 is FKBP_l derived from *Lelliottia amnigena.* The polypeptide consisting of the amino acid sequence of SEQ ID NO: 3 is FKBP_K derived from *Klebsiella quasipneumoniae*. The polypeptide consisting of the amino acid sequence of SEQ ID NO: 4 is FKBP_E derived from *Erwinia* sp. B116. The polypeptide consisting of the amino acid sequence of SEQ ID NO: 5 is FKBP_P derived from *Pantoea* sp. Nvir. The polypeptide consisting of any of the amino acid sequences of SEQ ID NOs: 1 to 6 is a polypeptide belonging to the FKBP family and has an isomerization activity of a prolyl bond. Among them, the polypeptide consisting of any of the amino acid sequences of SEQ ID NOs: 1 to 5 is a polypeptide belonging to a BCFK clade.

"An isomerization activity of a prolyl bond" means an activity to isomerize a cis-trans prolyl bond in a protein. The activity to isomerize a prolyl bond may be evaluated by use of a method well known in the art. The activity can be determined, for example, by measuring the refolding rate of ribonuclease T1 (RNase) by a polypeptide of interest (Marika Vitikainen et al., Journal of Biological Chemistry, 2004, 279 (18): 19302-19314).

The gene encoding heterologous FKBP is a polynucleotide encoding the heterologous FKBP mentioned above. The gene encoding heterologous FKBP is preferably a polynucleotide consisting of the nucleotide sequence of SEQ ID NO: 35 encoding FkpA, a polynucleotide consisting of the nucleotide sequence of SEQ ID NO: 40 encoding FKBP_l, a polynucleotide consisting of the nucleotide sequence of SEQ ID NO: 41 encoding FKBP_K, a polynucleotide consisting of the nucleotide sequence of SEQ ID NO: 42 encoding FKBP_E, a polynucleotide consisting of the nucleotide sequence of SEQ ID NO: 43 encoding FKBP_P, a polynucleotide consisting of the nucleotide sequence of SEQ ID NO: 46 encoding MJFKS, or a polynucleotide which consists of a nucleotide sequence having at least 80% identity to any of the nucleotide sequences of SEQ ID NOs: 35, 40 to 43, and 46 and encodes a polypeptide having an isomerization activity of a prolyl bond, more preferably the polynucleotide consisting of any of the nucleotide sequences of SEQ ID NOs: 35, 40 to 43, and 46.

The gene encoding heterologous FKBP can be prepared in accordance with a routine method. The gene encoding heterologous FKBP can be prepared, for example, by extracting genomic DNA by a routine method from a microbe or an organism originally producing the heterologous FKBP, or extracting RNA therefrom and synthesizing cDNA by reverse transcription. For example, the gene encoding FkpA (SEQ ID NO: 35) can be prepared from *Escherichia coli* (NBRC 3301) or the like. The microbe can be purchased from a public microbe collection organization. The gene encoding heterologous FKBP, obtained by the procedures described above, may be further subjected to site-direct mutagenesis to prepare a gene encoding heterologous FKBP and having a mutation. Alternatively, the gene encoding heterologous FKBP may be chemically synthesized on the basis of the amino acid sequence of the heterologous FKBP. The gene encoding heterologous FKBP may be codon-optimized depending on the species of the gram-positive bacterium as a host cell. Information on codons which are used by various organisms is available from Codon Usage Database ([www.kazusa.or.jp/codon/]).

The gene encoding heterologous FKBP may be operably linked to a control region. The "control region" is a region having a function of controlling intracellular expression of a gene located downstream thereof, preferably a function of constitutively expressing or highly expressing the gene located downstream thereof. More specifically, the control region may be defined as a region which resides upstream of a coding region of the gene and has a function of controlling the transcription of the coding region through interaction with RNA polymerase. Preferably, the control region in the present specification refers to a region of from approximately 200 to 600 nucleotides upstream of the coding region of the gene. The control region contains a transcription initiation control region and/or a translation initiation control region, or a region from the transcription initiation control region to the translation initiation control region. The transcription initiation control region is a region containing a promoter and a transcription initiation point, and the translation initiation control region is a site corresponding to a Shine-Dalgarno (SD) sequence which forms a ribosomal binding site together with a start codon (Shine, J., Dalgarno, L., Proc. Natl. Acad. Sci. USA., 1974, 71: 1342-1346).

Preferred examples of the control region include, but are not particularly limited to, control regions functioning in gram-positive bacteria, preferably bacteria of the genus *Bacillus,* for example, a control region of α-amylase gene, protease gene, aprE gene, or spoVG gene derived from bacteria of the genus *Bacillus,* a control region of cellulase gene of a *Bacillus* sp. KSM-S237 strain (JP-A-2000-210081), a control region of cellulase gene of a *Bacillus* sp. KSM-64 strain (JP-A-2011-10387), and a control region of kanamycin resistance gene or chloramphenicol resistance gene derived from *Staphylococcus aureus* (for both the genes, see JP-A-2009-089708). More preferred examples of the control region include promoters derived from bacteria of the genus *Bacillus,* for example, a promoter of the cellulase gene of the *Bacillus* sp. KSM-S237 strain (SEQ ID NO: 94), a promoter of the cellulase gene of the *Bacillus* sp. KSM-64 strain (SEQ ID NO: 95), and a promoter of *Bacillus subtilis* spoVG gene (SEQ ID NO: 96). Other preferred examples of the control region include promoters consisting of a nucleotide sequence having at least 90% identity to any of SEQ ID NOs: 94 to 96.

Preferably, the gene encoding heterologous FKBP is operably linked to a polynucleotide encoding a secretory signal peptide (referred to as a secretory signal sequence) which has a function of secreting expressed FKBP to the outside of cells from the viewpoint of improvement in the productivity of the protein of interest. Preferred examples of the secretory signal sequence include secretory signal sequences that function in gram-positive bacteria, preferably bacteria of the genus *Bacillus,* for example, secretory signal sequences derived from bacteria of the genus *Bacillus.* Preferred examples of the secretory signal sequences derived from bacteria of the genus *Bacillus* include a secretory signal sequence of the cellulase gene of the *Bacillus* sp. KSM-S237 strain (SEQ ID NO: 97), a secretory signal sequence of the cellulase gene of the *Bacillus* sp. KSM-64 strain (SEQ ID NO: 98), and a secretory signal sequence of *Bacillus subtilis* amylase gene amyE (SEQ ID NO: 99). Further examples of the secretory signal sequences derived from bacteria of the genus *Bacillus* include a nucleotide sequence which has at least 80% identity to any of SEQ ID NOs: 97 to 99 and has a function of secreting an expressed protein to the outside of cells. The sequence encoding heterologous FKBP to be linked to such a secretory signal sequence derived from a bacterium of the genus *Bacillus* may or may not contain a natural secretory signal sequence of the heterologous FKBP.

Thus, the gene encoding heterologous FKBP may contain an open reading frame (ORF) as well as a nucleotide sequence of an untranslated region (UTR). The gene may contain, for example, the aforementioned promoter and secretory signal sequence, and a terminator.

In the method of the present invention, the gene encoding the protein of interest may be a foreign gene encoding a heterologous protein (heterologous gene), may be an externally introduced gene encoding a homologously derived protein, or may be an endogenous gene encoding a protein which can be originally expressed by a host cell. The protein of interest is preferably a protein having at least one prolyl bond isomerization site. In this context, the prolyl bond isomerization site in the protein of interest is a site capable of being isomerized in the course of production of the protein of interest, i.e., a cis-prolyl binding site. The presence or absence and/or the number of the cis-prolyl binding site in the protein of interest can be confirmed with reference to the conformation of the protein of interest obtained by X-ray crystallography. The type of the protein of interest is not particularly limited. Examples of the protein of interest include desired proteins and enzymes involved in the synthesis of desired substances of interest and include antibody-related molecules, cytokines, hormones, other biologically active peptides, transporters, industrial enzymes such as protease, and metabolism-related enzymes.

Preferably, the protein of interest is an antibody-related molecule, more preferably a small-molecule antibody such as a Fab, a ScFv, or a VHH. Further more preferably, the protein of interest is selected from the group consisting of a Fab, a ScFv, and a VHH, further more preferably a Fab.

The gene encoding the protein of interest can be prepared in accordance with a routine method. The gene encoding the protein of interest can be prepared, for example, by extracting genomic DNA by a routine method from a microbe or an organism originally producing the protein of interest, or extracting RNA therefrom and synthesizing cDNA by reverse transcription. Alternatively, the gene encoding the protein of interest can be chemically synthesized on the basis of the amino acid sequence of the protein of interest. The gene encoding the protein of interest may be codon-optimized depending on the species of the gram-positive bacterium as a host cell.

The gene encoding the protein of interest may be operably linked to a control region. Preferably, the gene encoding the protein of interest is operably linked to a secretory signal sequence which allows an expressed protein of interest to be secreted to the outside of cells. Details of the control region and the secretory signal are the same as those mentioned about the gene encoding heterologous FKBP.

The gene encoding heterologous FKBP and the gene encoding the protein of interest may be introduced into the same gram-positive bacterium or may separately be introduced into different gram-positive bacteria. When the gene encoding heterologous FKBP and the gene encoding the protein of interest are separately introduced into different gram-positive bacteria, these gram-positive bacteria may be of the same species or different species; but are preferably of the same species from the viewpoint of culture efficiency. When the gene encoding heterologous FKBP and the gene encoding the protein of interest are separately introduced into different gram-positive bacteria, preferably, each of the genes is operably linked to a secretory signal sequence. More preferably, the gene encoding heterologous FKBP and the gene encoding the protein of interest are introduced into the same gram-positive bacterium.

The gram-positive bacterium as a host cell is not particularly limited as long as it can be used in protein production. Examples thereof include bacteria of the genus *Bacillus* such as *Bacillus subtilis, Bacillus licheniformis, Bacillus cereus, Bacillus thuringiensis,* and *Bacillus amyloliquefaciens,* and bacteria of the genus *Clostridium* such as *Clostridium butyricum.* Among them, a bacterium of the genus *Bacillus* is preferred, and *Bacillus subtilis* is more preferred.

The gram-positive bacterium may be a wild strain or may be a mutant strain prepared by a mutation. For example, a *Bacillus subtilis* strain deficient in at least one extracellular protease gene is preferably used as a mutant strain of *Bacillus subtilis* as the stain improves the productivity of the protein by effectively preventing extracellular degradation of the protein secreted to the outside of cells. In this context, the term "deficient" in extracellular protease means that the activity of extracellular protease is reduced in comparison to the activity in extracellular protease non-deficient cells (e.g., a wild strain). The cell deficient in extracellular protease (extracellular protease-deficient strain) can be prepared by partial or complete deletion or inactivation (so-called knock-down or knock-out, or the like) of the gene of the extracellular protease in a cell. Preferably, the extracellular protease-deficient strain has the activity of the extracellular protease reduced to 50% or less, more preferably 25% or less, based on the wild strain. The deficient extracellular protease gene in the extracellular protease-deficient *Bacillus subtilis* strain is preferably at least one gene, more preferably three genes, selected from the group consisting of aprE (BSORF DB gene position: BG10190), epr (BG10561), wprA (BG11846), mpr (BG10690), nprB (BG10691), bpr (BG10233), nprE (BG10448), vpr (BG10591) and aprX (BG12567), further more preferably all the genes. Preferred examples of the *Bacillus subtilis* mutant strain include an extracellular protease-deficient *Bacillus subtilis* strain Dpr9 (see JP-B-4485341). The *Bacillus subtilis* mutant strain may lack a sigma factor such as sigF (see JP-B-4336082), the deletion of recA involved in homologous recombination activity (see JP-B-6088282), or the like. *A Bacillus subtilis* mutant strain prepared by deleting one or both of sigF and recA in the Dpr9 strain can also be used.

The gene encoding heterologous FKBP, the gene encoding the protein of interest, or the gene encoding heterologous FKBP and the gene encoding the protein of interest can be introduced into the gram-positive bacterium as a host cell in accordance with a standard method. For example, the gene encoding heterologous FKBP can be integrated into the genome of the host gram-positive bacterial cell by introducing the gene encoding heterologous FKBP or a vector containing this gene into the host cell. The gene encoding the protein of interest can be integrated into the genome of the host gram-positive bacterial cell by introducing the gene encoding the protein of interest or a vector containing this gene into the host cell. The gene encoding heterologous FKBP and the gene encoding the protein of interest can be integrated into the genome of the host gram-positive bacterial cell by introducing the gene encoding heterologous FKBP or the vector containing this gene and the gene encoding the protein of interest or the vector containing this gene, or a vector containing both the gene encoding heterologous FKBP and the gene encoding the protein of interest into the host cell. Alternatively, an expression vector containing the gene encoding heterologous FKBP, an expression vector containing the gene encoding the protein of interest, the expression vector containing the gene encoding heterologous FKBP and the expression vector containing the gene encoding the protein of interest, or an expression vector containing both the gene encoding heterologous FKBP and the gene encoding the protein of interest may be introduced into the host gram-positive bacterial cell. In the case of introducing two genes or two vectors into the same host cell, the order of introduction thereof is not limited. Either of the genes or the vectors may be introduced first, or the genes or the vectors may be introduced at the same time.

For example, a transformation technique known in the art such as a competent cell method, an electroporation method, a protoplast method, a particle gun method, or a PEG method can be applied to the introduction of the genes or the vectors into gram-positive bacterial cells.

The vector containing the gene encoding heterologous FKBP, the vector containing the gene encoding the protein of interest, or the vector containing the gene encoding heterologous FKBP and the gene encoding the protein of interest can be constructed by inserting and linking the gene encoding heterologous FKBP and/or the gene encoding the protein of interest and optionally a control region or a secretory signal sequence into an arbitrary vector by a routine method. The type of the vector is not particularly limited and can be an arbitrary vector such as a plasmid, a phage, a phagemid, a cosmid, a virus, a YAC vector, or a shuttle vector. The vector is preferably a vector capable of being amplified in the host cell, more preferably an expression vector. Preferred examples of the vector include, but are not limited to: shuttle vectors such as pHA3040SP64, pHSP64R, and pASP64 (JP-B-3492935), pHY300PLK (expression vector capable of transforming both of E. coli and Bacillus subtilis; Jpn J Genet, 1985, 60: 235-243), pHY-S237 (JP-A-2014-158430), pAC3 (Nucleic Acids Res, 1988,16: 8732); and plasmids which can be used in the transformation of bacteria of the genus *Bacillus,* such as pUB110 (J Bacteriol, 1978, 134: 318-329) and pTA10607 (Plasmid, 1987, 18: 8-15). A plasmid derived from *E. coli* (e.g., pET22b(+), pBR322, pBR325, pUC57, pUC118, pUC119, pUC18, pUC19, or pBluescript) can also be used.

The method of the present invention involves culturing the gram-positive bacterium containing the gene encoding heterologous FKBP and the gene encoding the protein of interest, obtained by the procedures as described above, or culturing a mixture of the gram-positive bacterium containing the gene encoding heterologous FKBP and the gram-positive bacterium containing the gene encoding the protein of interest. The gram-positive bacterium can be cultured in accordance with a general gram-positive bacterium culture method. A medium for the culture of the gram-positive bacterium contains, for example, a carbon source and a nitrogen source necessary for the growth of the bacterium. Examples of the carbon source include glucose, dextran, soluble starch, sucrose, and methanol. Examples of the nitrogen source include ammonium salts, nitrates, amino acids, corn steep liquor, peptone, casein, meat extracts, soybean cake, and potato extracts. The medium may optionally contain other nutrients, for example, inorganic salts (e.g., sodium chloride, calcium chloride, sodium dihydrogen phosphate, and magnesium chloride), vitamins, and antibiotics (e.g., tetracycline, neomycin, kanamycin, spectinomycin, and erythromycin). Culture conditions, for example, a temperature, aeration and stirring conditions, pH of the medium, and a culture time may be appropriately selected depending on a bacterial species, traits, a culture scale, and the like.

The gram-positive bacterium is cultured so that the protein of interest is intracellularly expressed in the gram-positive bacterium. When the gene encoding the protein of interest is linked to a polynucleotide encoding a secretory signal peptide, the expressed protein of interest is secreted to the outside of the cell. The protein of interest produced by the method of the present invention can be collected from the culture solution by using, singly or in appropriate combinations, general methods for use in protein purification, for example, cell disruption, centrifugation, ammonium sulfate precipitation, gel chromatography, ion exchange chromatography, and affinity chromatography.

The method of the present invention allows the gram-positive bacterium to express heterologous FKBP during the expression of the protein of interest and thereby largely improves the productivity of the protein of interest in comparison to the expression of the protein of interest alone, as shown in Examples mentioned later. The method of the present invention largely improves the productivity of the protein of interest even in comparison to the case where PrsA reported to contribute to improvement in the productivity of the protein of interest in *Bacillus subtilis* (Patent Literature 2 and Non Patent Literatures 5 and 6) coexists during the expression of the protein of interest in the gram-positive bacterium. Thus, the method of the present invention can efficiently produce the protein of interest with high productivity using the gram-positive bacterium and achieves reduction in time and cost required for the production of the protein of interest.

Furthermore, the method of the present invention allows the gram-positive bacterium to secretory express heterologous FKBP during the expression of the protein of interest and thereby largely improves the productivity of the protein of interest in comparison to the expression of the heterologous FKBP in a state anchored to a cell membrane during the expression of the protein of interest. Proteins produced in *Bacillus subtilis* are known to be folded near a cell membrane (Non Patent Literature 4). It is totally unexpected to improve the productivity of the protein of interest when FKBP, which is capable of assisting in protein folding, is secretory expressed, rather than near a cell membrane.

The present invention also encompasses substances, production methods, use, methods, and the like given below as exemplary embodiments. However, the present invention is not limited to these embodiments.
[1] A method for producing a protein of interest, comprising: culturing a gram-positive bacterium comprising a gene encoding heterologous FKBP and a gene encoding the protein of interest; or culturing a mixture of a gram-positive bacterium comprising the gene encoding heterologous FKBP and a gram-positive bacterium comprising the gene encoding the protein of interest, preferably comprising culturing the gram-positive bacterium comprising the gene encoding heterologous FKBP and the gene encoding the protein of interest.
[2] A method for improving productivity of a protein of interest, comprising: culturing a gram-positive bacterium comprising a gene encoding heterologous FKBP and a gene encoding the protein of interest; or culturing a mixture of a gram-positive bacterium comprising the gene encoding heterologous FKBP and a gram-positive bacterium comprising the gene encoding the protein of interest, preferably comprising culturing the gram-positive bacterium comprising the gene encoding heterologous FKBP and the gene encoding the protein of interest.
[3] The method according to [1] or [2], wherein the heterologous FKBP is a polypeptide which belongs to a BCFK clade and has an isomerization activity of a prolyl bond, a polypeptide consisting of the amino acid sequence of SEQ ID NO: 6, or a polypeptide which consists of an amino acid sequence having at least 80% identity to the amino acid sequence of SEQ ID NO: 6 and has an isomerization activity of a prolyl bond, preferably the polypeptide which belongs to a BCFK clade and has an isomerization activity of a prolyl bond or the polypeptide consisting of the amino acid sequence of SEQ ID NO: 6.
[4] The method according to [1] or [2], wherein the heterologous FKBP is a polypeptide consisting of any of the amino acid sequences of SEQ ID NOs: 1 to 6, or a polypeptide which consists of an amino acid sequence having at least 80% identity to any of the amino acid sequences of SEQ ID NOs: 1 to 6 and has an isomerization activity of a prolyl bond, preferably the polypeptide consisting of any of the amino acid sequences of SEQ ID NOs: 1 to 6.
[5] The method according to [1] or [2], wherein the gene encoding heterologous FKBP is a polynucleotide consisting of any of the nucleotide sequences of SEQ ID NOs: 35, 40 to 43, and 46, or a polynucleotide which consists of a nucleotide sequence having at least 80% identity to any of the nucleotide sequences of SEQ ID NOs: 35, 40 to 43, and 46 and encodes a polypeptide having an isomerization activity of a prolyl bond, preferably the polynucleotide consisting of any of the nucleotide sequences of SEQ ID NOs: 35, 40 to 43, and 46.
[6] The method according to any one of [1] to [5], wherein the gene encoding heterologous FKBP is operably linked to a polynucleotide encoding a secretory signal peptide.
[7] The method according to any one of [1] to [6], wherein the gram-positive bacterium is a bacterium of the genus *Bacillus,* preferably *Bacillus subtilis* or a mutant strain thereof.
[8] The method according to any one of [1] to [6], wherein the gram-positive bacterium is *Bacillus subtilis* with at least one of extracellular protease gene deleted or inactivated, preferably *Bacillus subtilis* with at least one extracellular protease gene deleted or inactivated, the gene being selected from the group consisting of aprE, epr, wprA, mpr, nprB, bpr, nprE, vpr and aprX, more preferably *Bacillus subtilis* with aprE, epr, wprA, mpr, nprB, bpr, nprE, vpr, and aprX deleted or inactivated.
[9] The method according to any one of [1] to [8], wherein the protein of interest has at least one prolyl bond isomerization site.
[10] The method according to any one of [1] to [9], wherein the protein of interest is an antibody-related molecule, preferably selected from the group consisting of a Fab, a ScFv, and a VHH, more preferably a Fab.
[11] The method according to any one of [1] to [10], further comprising collecting the protein of interest from the cultures.
[12] A gram-positive bacterium comprising a gene encoding heterologous FKBP, or the gene encoding heterologous FKBP and a gene encoding a protein of interest.
[13] The gram-positive bacterium according to [12], wherein the heterologous FKBP is a polypeptide which belongs to a BCFK clade and has an isomerization activity of a prolyl bond, a polypeptide consisting of the amino acid sequence of SEQ ID NO: 6, or a polypeptide which consists of an amino acid sequence having at least 80% identity to the amino acid sequence of SEQ ID NO: 6 and has an isomerization activity of a prolyl bond, preferably the polypeptide which belongs to a BCFK clade and has an isomerization activity of a prolyl bond or the polypeptide consisting of the amino acid sequence of SEQ ID NO: 6.
[14] The gram-positive bacterium according to [12], wherein the heterologous FKBP is a polypeptide consisting of any of the amino acid sequences of SEQ ID NOs: 1 to 6, or a polypeptide which consists of an amino acid sequence having at least 80% identity to any of the amino acid sequences of SEQ ID NOs: 1 to 6 and has an isomerization activity of a prolyl bond, preferably the polypeptide consisting of any of the amino acid sequences of SEQ ID NOs: 1 to 6.
[15] The gram-positive bacterium according to [12], wherein the gene encoding heterologous FKBP is a polynucleotide consisting of any of the nucleotide sequences of SEQ ID NOs: 35, 40 to 43, and 46, or a polynucleotide which consists of a nucleotide sequence having at least 80% identity to any of the nucleotide sequences of SEQ ID NOs: 35, 40 to 43, and 46 and encodes a polypeptide having an isomerization activity of a prolyl bond, preferably the polynucleotide consisting of any of the nucleotide sequences of SEQ ID NOs: 35, 40 to 43, and 46.
[16] The gram-positive bacterium according to any one of [12] to [15], wherein the gene encoding heterologous FKBP is operably linked to a polynucleotide encoding a secretory signal peptide.
[17] The gram-positive bacterium according to any one of [12] to [16], wherein the gram-positive bacterium is a bacterium of the genus *Bacillus,* preferably *Bacillus subtilis* or a mutant strain thereof.
[18] The gram-positive bacterium according to any one of [12] to [16], wherein the gram-positive bacterium is *Bacillus subtilis* with at least one of extracellular protease gene deleted or inactivated, preferably *Bacillus subtilis* with at least one extracellular protease gene deleted or inactivated, the gene being selected from the group consisting of aprE, epr, wprA, mpr, nprB, bpr, nprE, vpr and aprX, more preferably *Bacillus subtilis* with aprE, epr, wprA, mpr, nprB, bpr, nprE, vpr, and aprX deleted or inactivated.
[19] The gram-positive bacterium according to any one of [12] to [18], wherein the protein of interest has at least one prolyl bond isomerization site.
[20] The gram-positive bacterium according to any one of [12] to [19], wherein the protein of interest is an antibody-related molecule, preferably selected from the group consisting of a Fab, a ScFv, and a VHH, more preferably a Fab.

### Examples

Hereinafter, the present invention will be described further specifically with reference to Examples.
However, the technical scope of the present invention is not limited to these Examples.

### Material and method

### (1) Host bacterial strain

The host *Bacillus subtilis* was a derivative strain of a *Bacillus subtilis* 168 strain. A sigF gene-deficient strain (Dpr9ΔsigF) was prepared from an extracellular protease-deficient *Bacillus subtilis* strain Dpr9 (see JP-B-4485341; deficient in extracellular protease epr, wprA, mpr, nprB, bpr, nprE, vpr, aprE, and aprX) in accordance with a method described in JP-B-4336082.

### (2) Medium

LB medium: 1% Bacto^{™} Tryptone, 0.5% Bacto^{™} Yeast Extract, and 1% sodium chloride. A plate medium was supplemented with 1.5% agar. Tetracycline (50 ppm) was added, if necessary.

DM3 medium: 1% CMC (Kanto Chemical Co., Inc.), 0.5% Bacto^{™} Casamino Acids, 0.5% Bacto^{™} Yeast Extract, 8.1% disodium succinate·6H₂O, 0.35% dipotassium hydrogen phosphate, 0.15% potassium dihydrogen phosphate, 0.5% glucose, 20 mM magnesium chloride, 0.01% BSA, and 50 ppm tetracycline. A plate medium was supplemented with 1% agar.

2×L-mal medium: 2% Bacto^{™} Tryptone, 1% Bacto^{™} Yeast Extract, 1% sodium chloride, 7.5% maltose monohydrate, 7.5 ppm manganese sulfate, and 15 ppm tetracycline.

### Example 1 Increased production of protein by PPIase expression

### 1-1. Construction of plasmid for PPIase expression

A plasmid sequence was amplified by PCR using a recombinant plasmid pHY-S237 prepared on the basis of pHY300PLK (JP-A-2014-158430) as a template, a primer set of SEQ ID NO: 7 and SEQ ID NO: 8, and PrimeSTAR Max DNA polymerase (Takara Bio Inc.). Promoter DNA derived from spoVG gene was amplified by PCR using the genome of the 168 strain as a template and a primer set of SEQ ID NO: 9 and SEQ ID NO: 10. The obtained promoter DNA was incorporated into the plasmid sequence using In-Fusion HD Cloning Kit (Takara Bio Inc.). A cellulase sequence was removed from the obtained plasmid sequence by PCR using a primer set of SEQ ID NO: 11 and SEQ ID NO: 12. A plasmid sequence was amplified by PCR using the obtained plasmid as a template and a primer set of SEQ ID NO: 11 and SEQ ID NO: 13. Secretory signal DNA was amplified by PCR using the genome of the 168 strain as a template and a primer set of SEQ ID NO: 14 and SEQ ID NO: 15. The obtained secretory signal DNA was incorporated into the plasmid sequence using In-Fusion HD Cloning Kit (Takara Bio Inc.) to construct a plasmid for PPIase expression.

### 1-2. Design of plasmid for PPIase expression

A plasmid sequence was amplified by PCR using the plasmid for PPIase expression constructed in 1-1 as a template, a primer set of SEQ ID NO: 11 and SEQ ID NO: 13, and PrimeSTAR Max DNA polymerase (Takara Bio Inc.). DNA encoding PrsA of SEQ ID NO: 18 was amplified (SEQ ID NO: 19) by PCR using the genome of the 168 strain as a template and a primer set of SEQ ID NO: 16 and SEQ ID NO: 17. The obtained DNA encoding PrsA was incorporated into the plasmid sequence using In-Fusion HD Cloning Kit (Takara Bio Inc.) to construct a plasmid for PrsA expression. A plasmid sequence was amplified by PCR using the plasmid for PPIase expression constructed in 1-1 as a template, a primer set of SEQ ID NO: 13 and SEQ ID NO: 20, and PrimeSTAR Max DNA polymerase (Takara Bio Inc.). DNA encoding each of SurA (SEQ ID NO: 29), PpiA (SEQ ID NO: 30), Tig (SEQ ID NO: 31), and FkpA (SEQ ID NO: 1) was amplified (SEQ ID NOs: 32 to 35) by PCR using the genome of *E. coli* DH5α as a template and a primer set of SEQ ID NO: 21 and SEQ ID NO: 22, primer set of SEQ ID NO: 23 and SEQ ID NO: 24, primer set of SEQ ID NO: 25 and SEQ ID NO: 26, and primer set of SEQ ID NO: 27 and SEQ ID NO: 28, respectively. The obtained DNA encoding PPIase was incorporated into the plasmid sequence using In-Fusion HD Cloning Kit (Takara Bio Inc.) to construct each plasmid for SurA, PpiA, Tig, or FkpA expression. Each of nucleotide sequences of SEQ ID NOs: 40 to 48 encoding the amino acid sequences of FKBP_l (SEQ ID NO: 2), FKBP_K (SEQ ID NO: 3), FKBP_E (SEQ ID NO: 4), FKBP_P (SEQ ID NO: 5), FKBP2 (SEQ ID NO: 36), FK153 (SEQ ID NO: 37), MJFKS (SEQ ID NO: 6), FKBP52 (SEQ ID NO: 38), and CyP40 (SEQ ID NO: 39) were synthesized in a form incorporated between regions of SEQ ID NO: 49 and SEQ ID NO: 50 in the plasmid for PPIase expression constructed in 1-1 by GenPlus Cloning from GeneScript Japan Inc. to give each plasmid for FKBP_l, FKBP_K, FKBP_E, FKBP_P, FKBP2, FK153, MJFKS, FKBP52, or CyP40 expression.

### 1-3. Construction of Fab-expressing strain

A DNA fragment (A) containing a ybdO gene region and a DNA fragment (B) containing a ybxG gene region were each amplified by PCR using the genome of the 168 strain as a template and a primer set of SEQ ID NO: 51 and SEQ ID NO: 52 and primer set of SEQ ID NO: 53 and SEQ ID NO: 54, respectively. A chloramphenicol resistance gene region (C) was amplified by PCR using plasmid pC194 DNA as a template and a primer set of SEQ ID NO: 55 and SEQ ID NO: 56. A nucleotide sequence of SEQ ID NO: 59 capable of causing operon expression of Fab_c (certolizumab Fab) consisting of a heavy chain and a light chain of SEQ ID NO: 57 and SEQ ID NO: 58 was synthesized in a form incorporated between regions of SEQ ID NO: 60 and SEQ ID NO: 50 in the plasmid for PPIase expression constructed in 1-1 by GenPlus Cloning from GenScript Japan Inc. to give a plasmid for Fab_c expression. A DNA fragment (D) containing a Fab_c expression region was amplified by PCR using the plasmid for Fab_c expression as a template and a primer set of SEQ ID NO: 61 and SEQ ID NO: 62. These four fragments (A), (B), (C), and (D) were mixed and used as a template in SOE-PCR using a primer set of SEQ ID NO: 51 and SEQ ID NO: 54 so that the four fragments were linked to give a DNA fragment for genomic integration of the Fab_c expression region. The *Bacillus subtilis* Dpr9ΔsigF strain was transformed by the competent cell method using the obtained DNA fragment for genomic integration of the Fab_c expression region. After transformation, a colony grown on LB agar medium containing chloramphenicol (10 µg/mL) was separated as a transformant. The bacterial strain thus obtained was designated as a Fab_c-producing strain.

### 1-4. Introduction of plasmid for PPIase expression into Bacillus subtilis

The plasmid for PPIase expression constructed in 1-2 was introduced into the Fab_c-producing strain by the following protoplast method: a glycerol stock of *Bacillus subtilis* was inoculated to 1 mL of LB liquid medium and cultured overnight at 210 rpm at 30°C with shaking. On the next day, 10 µL of this culture solution was inoculated to 1 mL of fresh LB liquid medium and cultured at 210 rpm at 37°C for approximately 2 hours with shaking. This culture solution was collected into a 1.5 mL tube and centrifuged at 12 000 rpm for 5 minutes, and pellets obtained by the removal of the supernatant were suspended in 500 µL of SMMP containing 4 mg/mL Lysozyme (manufactured by Sigma-Aldrich Co., LLC) and incubated at 37°C for 1 hour. Subsequently, the incubated suspension was centrifuged at 3 500 rpm for 10 minutes, and pellets obtained by the removal of the supernatant were suspended in 400 µL of SMMP. 33 µL of this suspension was mixed with each plasmid, and 100 µL of 40% PEG was further added to the mixture, which was then vortexed. 350 µL of SMMP was added to this solution, and the mixture was mixed by inversion, shaken at 210 rpm at 30°C for 1 hour, then spread in the whole amount over a DM3 agar medium plate, and incubated at 30°C for 2 to 3 days.

### 1-5. Fab production

The recombinant *Bacillus subtilis* prepared in 1-4 was inoculated to 500 µL of LB medium containing 50 ppm tetracycline and cultured overnight at 30°C in a 96-well plate to prepare a preculture solution. 1% of the preculture solution was inoculated to 800 µL of 2×L-mal medium containing 15 ppm tetracycline and cultured at 30°C for 72 hours in a 96-well plate. At the completion of culture, the culture solution was centrifuged at 3 000 rpm at 4°C for 20 minutes, and the supernatant was collected.

### 1-6. Quantification of amount of Fab produced by ELISA

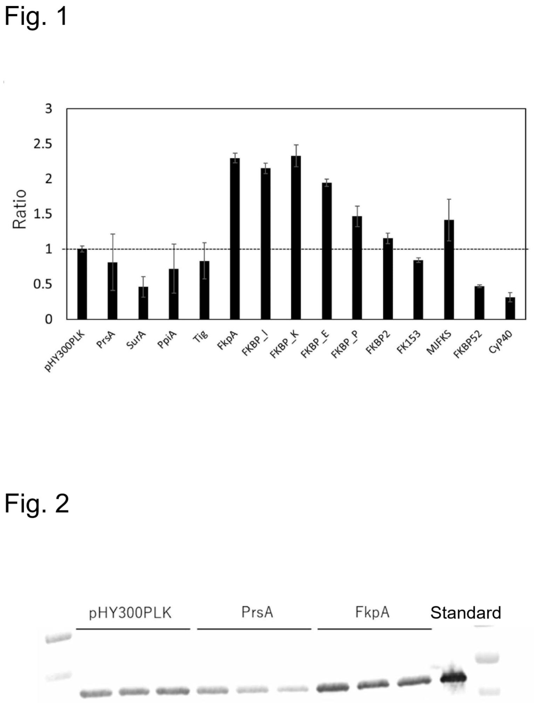

100 µL of 1 µg/mL TNF-α (FUJIFILM Wako Pure Chemical Corp.) was added to each well of F96 Cert.Maxisorp Nunc-Immuno^{™} Plate (Thermo Fisher Scientific Inc.), which was then sealed and then left standing overnight at 4°C. After removal of TNF-α which was not adsorbed to the well, 200 µL of PBST (PBS containing 0.1% (v/v) Tween 20) was added thereto and immediately removed. This washing operation was performed three times. PBST containing 2% skimmed milk was added thereto, and the plate was incubated at room temperature for 1 hour. After removal of PBST containing skimmed milk, 200 µL of PBST was added thereto and immediately removed. This washing operation was performed three times. The culture supernatant collected in 1-5 was diluted 50-fold with PBST and further diluted 10-fold with PBST containing 2% skimmed milk. The same dilution operation as above was carried out as to dilution series of Certolizumab Recombinant Human Monoclonal Antibody (Invitrogen Corp.). 100 µL of the solution thus diluted was added to each TNF-α-immobilized well and incubated at room temperature for 1 hour. After removal of the diluted solution, 200 µL of PBST was added thereto and immediately removed. This washing operation was performed three times. The detection antibody used was Goat anti-Human IgG (H+L) Secondary Antibody, HRP (Invitrogen Corp.). The detection antibody was diluted at 1/5 000 with PBST. 100 µL of the detection antibody was added to each well and incubated at room temperature for 1 hour. After removal of the detection antibody, 200 µL of PBST was added thereto and immediately removed. This washing operation was performed three times. A chromogenic substrate was prepared by dissolving OPD tablets (Thermo Fisher Scientific Inc.) in Stable Peroxide Substrate Buffer (Thermo Fisher Scientific Inc.). 100 µL of the chromogenic substrate was added to each well and incubated for 10 minutes in the dark. 100 µL of 0.5 mol/L sulfuric acid was added to each well, followed by the measurement of absorbance at 490 nm using a microplate reader. The amount of Fab_c produced was calculated on the basis of the dilution series concentrations of Certolizumab Recombinant Human Monoclonal Antibody (Invitrogen Corp.) and indicated as a relative value to the amount calculated for the Fab_c-producing strain harboring pHY300PLK (Fig. 1). As a result, large improvement in the amount of Fab_c produced was confirmed in the Fab_c-producing strain carrying the plasmid for FkpA, FKBP_l, FKBP_K, FKBP_E, FKBP_P, or MJFKS expression. The amount of Fab_c produced was reduced in the Fab_c-producing strain carrying the plasmid for PrsA (i.e., an endogenous factor of *Bacillus subtilis)* expression. A Fab_c has a prolyl bond isomerization site, specifically, a cis prolyl bond.

### 1-7. Confirmation of Fab production by Western blotting

The culture supernatant obtained in 1-5 and Laemmli Sample Buffer (Bio-Rad Laboratories, Inc.) were mixed in equal amounts and then heat-treated at 99°C for 5 minutes to prepare a sample. The gel used was Any kD^{™} Mini-PROTEAN TGX^{™} Precast Gel (Bio-Rad Laboratories, Inc.). 5 µL of the sample was applied to each well and then electrophoresed at 210 V for 5 minutes. Precision Plus Blue standard (Bio-Rad Laboratories, Inc.) was used as a molecular weight marker, and Certolizumab Recombinant Human Monoclonal Antibody (Invitrogen Corp.) was also electrophoresed as a standard at the same time therewith. Proteins were transferred from the SDS-PAGE gel to a PVDF membrane using Trans-Blot Turbo Mini PVDF Transfer Packs (Bio-Rad Laboratories, Inc.) and Trans-Blot Turbo System (Bio-Rad Laboratories, Inc.). The antibody used was Goat anti-Human IgG (H+L) Secondary Antibody, HRP (Invitrogen Corp.), and iBind Western System (Invitrogen Corp.) was used in antibody reaction. The protein of interest was detected using 1-Step Ultra TMB-Blotting Solution (Thermo Fisher Scientific Inc.) (Fig. 2). As in the quantification by ELISA shown in 1-6, improved productivity was confirmed in the Fab_c-producing strain carrying the plasmid for FkpA expression in comparison to the Fab_c-producing strain harboring pHY300PLK. Reduced productivity was also confirmed in the Fab_c-producing strain carrying the plasmid for PrsA expression in comparison to the Fab_c-producing strain harboring pHY300PLK.

### Example 2 Test on domain necessary for increased production

### 2-1. Design of plasmid for expression of each domain

A C-terminal region or an N-terminal region of FkpA was removed by PCR using the plasmid for FkpA expression constructed in 1-2 as a template, a primer set of SEQ ID NO: 63 and SEQ ID NO: 64 and a primer set of SEQ ID NO: 65 and SEQ ID NO: 66, and PrimeSTAR Max DNA polymerase (Takara Bio Inc.) to give a plasmid for FkpA_N expression capable of causing expression of the N-terminal region (SEQ ID NO: 67) of FkpA and a plasmid for FkpA_C expression capable of causing expression of the C-terminal region (SEQ ID NO: 68) of FkpA.

### 2-2. Introduction of plasmid for PPIase expression into Bacillus subtilis

The plasmid for expression of each domain constructed in 2-1 was introduced into the Fab_c-producing strain by the protoplast method shown in 1-4.

### 2-3. Fab production

The recombinant *Bacillus subtilis* prepared in 2-2 was inoculated to 500 µL of LB medium containing 50 ppm tetracycline and cultured overnight at 30°C in a 96-well plate to prepare a preculture solution. 1% of the preculture solution was inoculated to 800 µL of 2×L-mal medium containing 15 ppm tetracycline and cultured at 30°C for 72 hours in a 96-well plate. At the completion of culture, the culture solution was centrifuged at 3 000 rpm at 4°C for 20 minutes, and the supernatant was collected.

### 2-4. Quantification of amount of Fab produced by ELISA

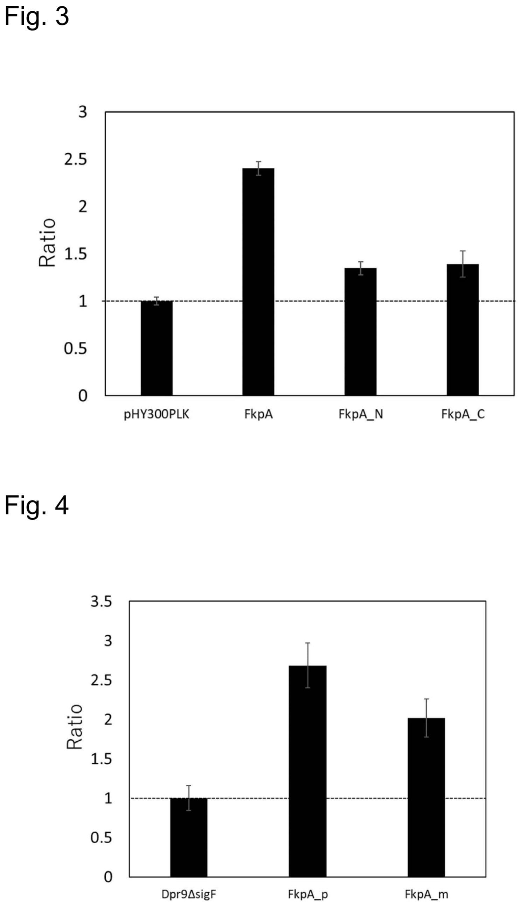

The amount of Fab_c produced was quantified by the ELISA shown in 1-6. The amount of Fab_c produced was calculated on the basis of the dilution series concentrations of Certolizumab Recombinant Human Monoclonal Antibody (Invitrogen Corp.) and indicated as a relative value to the amount calculated for the Fab_c-producing strain harboring pHY300PLK (Fig. 3). As a result, the expression of both the N-terminal region and the C-terminal region of FkpA was confirmed to be necessary for producing a large productivity-improving effect in the *Bacillus subtilis* production system.

### Example 3 Test on protein production-increasing effect depending on localization site of PPIase

### 3-1. Construction of plasmid for Fab expression

A plasmid sequence was amplified by PCR using a recombinant plasmid pHY-S237 (JP-A-2014-158430) prepared on the basis of pHY300PLK as a template, a primer set of SEQ ID NO: 7 and SEQ ID NO: 8, and PrimeSTAR Max DNA polymerase (Takara Bio Inc.). Promoter DNA derived from spoVG gene was amplified by PCR using the genome of the 168 strain as a template and a primer set of SEQ ID NO: 9 and SEQ ID NO: 10. The obtained promoter DNA was incorporated into the plasmid sequence using In-Fusion HD Cloning Kit (Takara Bio Inc.). A plasmid sequence was amplified by PCR using the obtained plasmid as a template and a primer set of SEQ ID NO: 11 and SEQ ID NO: 13. A nucleotide sequence of SEQ ID NO: 71 capable of causing operon expression of Fab_l (Fab against lysozyme) consisting of a heavy chain and a light chain of SEQ ID NO: 69 and SEQ ID NO: 70 was artificially synthesized at Thermo Fisher Scientific Inc. and incorporated into the plasmid sequence using In-Fusion HD Cloning Kit (Takara Bio Inc.) to construct a plasmid for Fab_l expression.

### 3-2. Construction of PPIase-expressing strain

A DNA fragment (A) containing a bglP gene region, a DNA fragment (B) containing a yxxE gene region, and promoter DNA (C) derived from spoVG gene were each amplified by PCR using the genome of the 168 strain as a template and a primer set of SEQ ID NO: 72 and SEQ ID NO: 73, a primer set of SEQ ID NO: 74 and SEQ ID NO: 75, and a primer set of SEQ ID NO: 76 and SEQ ID NO: 77, respectively. A chloramphenicol resistance gene region (D) was amplified by PCR using plasmid pC194 DNA as a template and a primer set of SEQ ID NO: 55 and SEQ ID NO: 56. DNA encoding FkpA linked to a secretory signal or a signal for anchoring the protein onto membranes was amplified ((E) or (E')) by PCR using the genome of *E*. *coli* DH5α as a template and a primer set of SEQ ID NO: 78 and SEQ ID NO: 79 and a primer set of SEQ ID NO: 79 and SEQ ID NO: 80, respectively. These five fragments (A), (B), (C), (D), and (E) or (A), (B), (C), (D), and (E') were mixed and used as a template in SOE-PCR using a primer set of SEQ ID NO: 72 and SEQ ID NO: 75 so that the five fragments were linked to give a DNA fragment for genomic integration of FkpA to be secretory expressed or expressed by anchoring to membranes. The *Bacillus subtilis* Dpr9ΔsigF strain was transformed by the competent cell method using the obtained DNA fragment for genomic integration. After transformation, a colony grown on LB agar medium containing chloramphenicol (10 µg/mL) was separated as a transformant. The bacterial strain capable of secretory expressing FkpA thus obtained was designated as an FkpA_p strain, and the bacterial strain capable of expressing FkpA anchored to membranes was designated as an FkpA_m strain.

### 3-3. Introduction of plasmid for Fab expression into Bacillus subtilis

The plasmid for Fab_l expression constructed in 3-1 was introduced into the FkpA_p strain and the FkpA_m strain by the protoplast method shown in 1-4.

### 3-4. Fab production

The recombinant *Bacillus subtilis* prepared in 3-3 was inoculated to 500 µL of LB medium containing 50 ppm tetracycline and cultured overnight at 30°C in a 96-well plate to prepare a preculture solution. 1% of the preculture solution was inoculated to 800 µL of 2×L-mal medium containing 15 ppm tetracycline and cultured at 30°C for 72 hours in a 96-well plate. At the completion of culture, the culture solution was centrifuged at 3 000 rpm at 4°C for 20 minutes, and the supernatant was collected.

### 3-5. Quantification of amount of Fab produced by ELISA

100 µL of 100 µg/mL Lysozyme, from Egg White (FUJIFILM Wako Pure Chemical Corp.) was added to each well of F96 Cert.Maxisorp Nunc-Immuno^{™} Plate (Thermo Fisher Scientific Inc.), which was then sealed and then left standing overnight at 4°C. After removal of Lysozyme which was not adsorbed to the well, 200 µL of PBST (PBS containing 0.1% (v/v) Tween 20) was added thereto and immediately removed. This washing operation was performed three times. PBST containing 2% skimmed milk was added thereto, and the plate was incubated at room temperature for 1 hour. After removal of PBST containing skimmed milk, 200 µL of PBST was added thereto and immediately removed. This washing operation was performed three times. The culture supernatant collected in 3-4 was diluted 10-fold with PBST containing 2% skimmed milk. 100 µL of the solution thus diluted was added to each Lysozyme-immobilized well and incubated at room temperature for 1 hour. After removal of the diluted solution, 200 µL of PBST was added thereto and immediately removed. This washing operation was performed three times. The detection antibody used was a Mouse IgG Fab Antibody:HRP (BioValley). The detection antibody was diluted at 1/5 000 with PBST. 100 µL of the detection antibody was added to each well and incubated at room temperature for 1 hour. After removal of the detection antibody, 200 µL of PBST was added thereto and immediately removed. This washing operation was performed three times. A chromogenic substrate was prepared by dissolving OPD tablets (Thermo Fisher Scientific Inc.) in Stable Peroxide Substrate Buffer (Thermo Fisher Scientific Inc.). 100 µL of the chromogenic substrate was added to each well and incubated for 20 minutes in the dark. 100 µL of 0.5 mol/L sulfuric acid was added to each well, followed by the measurement of absorbance at 490 nm using a microplate reader. The obtained absorbance was indicated as a relative value to the absorbance for the Dpr9ΔsigF strain harboring the plasmid for Fab_l expression (Fig. 4). As a result, although *Bacillus subtilis* is known to fold proteins near a cell membrane, it was revealed that the secretory expression of FkpA contributes more to increased production of the protein of interest than the expression of FkpA anchored to the membrane. A Fab_l has a prolyl bond isomerization site, specifically, a cis prolyl bond.

### Example 4 Increased production of protein by PPIase expression

### 4-1. Construction of plasmid for RNaseT1 expression

A nucleotide sequence of SEQ ID NO: 101 encoding the amino acid sequence of RNaseT1 (SEQ ID NO: 100) was synthesized in a form incorporated between regions of SEQ ID NO: 49 and SEQ ID NO: 50 in the plasmid for PPIase expression constructed in 1-1 by GenPlus Cloning from GenScript Japan Inc. to give a plasmid for RNaseT1 expression.

### 4-2. Introduction of plasmid for RNaseT1 expression into PPIase-expressing strain

The plasmid for RNaseT1 expression was introduced into the *Bacillus subtilis* Dpr9ΔsigF strain and the FkpA_p strain constructed in 3-2 by the protoplast method shown in 1-4.

### 4-3. RNaseT1 production

The recombinant *Bacillus subtilis* prepared in 4-2 was inoculated to 500 µL of LB medium containing 50 ppm tetracycline and cultured overnight at 30°C in a 96-well plate to prepare a preculture solution. 1% of the preculture solution was inoculated to 800 µL of 2×L-mal medium containing 15 ppm tetracycline and cultured at 30°C for 72 hours in a 96-well plate. At the completion of culture, the culture solution was centrifuged at 3 000 rpm at 4°C for 20 minutes, and the supernatant was collected.

### 4-4. Quantification of amount of RNaseT1 produced by SDS-PAGE

The culture supernatant collected in 4-3 and Laemmli Sample Buffer (Bio-Rad Laboratories, Inc.) were mixed in equal amounts and then heat-treated at 99°C for 5 minutes to prepare a sample. The gel used was Mini-PROTEIN TGX Stain-Free (Bio-Rad Laboratories, Inc.). 5 µL of the sample was applied to each well and then electrophoresed at 210 V for 25 minutes. Precision Plus protein Unstained standard (Bio-Rad Laboratories, Inc.) was used as a molecular weight marker. A protein band was detected using ChemiDoc MP Imaging System. The detected protein was quantified from a calibration curve prepared on the basis of a lysozyme standard (Sigma-Aldrich Co., LLC) (Table 1). As a result, the expression of PPIase was confirmed to largely improve the amount of RNaseT1 produced. RNaseT1 has a prolyl bond isomerization site, specifically, a cis prolyl bond.

**[Table 1]**

| | Amount of RNaseT1 produced (g/L) |
|---|---|
| Dpr9 ΔsigF strain | 0.15 |
| FkpA_p strain | 1.29 |

### Reference Example 1 Identification of PPIase sequence group belonging to BCFK clade using HMM profile Phylogenetic analysis of FKBP

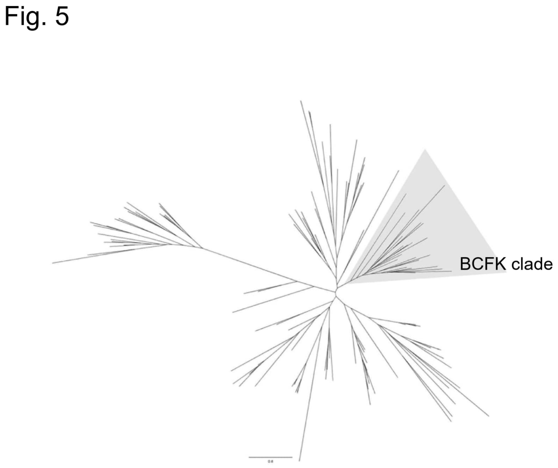

Phylogenetic analysis was conducted using an FKBP sequence group registered as seed sequences of the PF00254 family in Pfam, which is shown in Table 2, plus an FkpA sequence of SEQ ID NO: 81. Multiple alignment was constructed using default parameters of MAFFT v7.471. Then, a conservative region was extracted using trimAl v1.4. rev15, and an evolutionary model was selected using ModelTest-NG v0.1.7. IQ-TREE multicore version 2.0.3 was used in the phylogenetic analysis, and LG+G4+F was assigned to the evolutionary model. Fig. 5 illustrates a phylogenetic tree visualized using Figtree (http://tree.bio.ed.ac.uk/software/FigTree/). Table 3 shows Uniprot numbers of the FKBP sequence group belonging to the BCFK clade in Fig. 5.

**[Table 2]**

| | | | | | | | |
|---|---|---|---|---|---|---|---|
| 1 | Q9FJL3.1 | 32 | Q9PQE4.1 | 63 | Q38931.2 | 94 | Q9HYX8.1 |
| 2 | A0A3B6C680.1 | 33 | Q8XKK0.1 | 64 | Q13451.2 | 95 | Q9A2C9.1 |
| 3 | U3D5N6.1 | 34 | Q97FT6.1 | 65 | P27124.3 | 96 | Q9A4N1.1 |
| 4 | Q9VL78.1 | 35 | Q8RC26.1 | 66 | Q9RTC6.1 | 97 | A0KGZ9.1 |
| 5 | O45418.1 | 36 | Q55511.1 | 67 | P20081.2 | 98 | 083834.1 |
| 6 | Q43207.1 | 37 | Q935Z3.1 | 68 | O42993.1 | 99 | Q9KLT0.1 |
| 7 | Q14318.2 | 38 | O8YQX9.1 | 69 | P0CP94.1 | 100 | Q8PAI1.1 |
| 8 | Q9VYT2.1 | 39 | P32472.1 | 70 | P48375.2 | 101 | Q9KV04.1 |
| 9 | Q9LDC0.1 | 40 | I1JM68.1 | 71 | Q8MTB4.1 | 102 | Q8XZ41.1 |
| 10 | Q7DMA9.2 | 41 | O46638.2 | 72 | Q62658.3 | 103 | Q9RJ63.1 |
| 11 | Q91XW8.1 | 42 | O54998.1 | 73 | H0ZSI4.1 | 104 | Q9RJ62.1 |
| 12 | Q9W1I9.1 | 43 | A0A0K0J7U9.1 | 74 | P28870.2 | 105 | P30417.2 |
| 13 | P26623.3 | 44 | Q95Q60.1 | 75 | A0A0N4TXG0.1 | 106 | Q944B0.1 |
| 14 | Q9HVM6.1 | 45 | O16309.1 | 76 | Q9U2Q8.1 | 107 | Q9LYR5.1 |
| 15 | Q8TLA2.1 | 46 | Q23338.2 | 77 | Q8Y1A6.1 | 108 | Q8LB65.1 |
| 16 | Q8TLA1.1 | 47 | P91180.1 | 78 | Q8P8Z0.1 | 109 | Q9LDY5.1 |
| 17 | P75454.1 | 48 | A0A669CFJ3.1 | 79 | P0A0W2.1 | 110 | Q9SR70.1 |
| 18 | P57980.1 | 49 | O61826.2 | 80 | P73037.1 | 111 | Q9LM71.2 |
| 19 | Q9KQS5.1 | 50 | Q96AY3.1 | 81 | Q8YZA2.1 | 112 | Q9FLB3.1 |
| 20 | Q912U2.1 | 51 | Q61576.2 | 82 | Q9HXS4.1 | 113 | Q93ZG9.1 |
| 21 | Q9JZ37.1 | 52 | G5E7J9.1 | 83 | Q9SCY2.2 | 114 | F4J9Q6.1 |
| 22 | Q8XYP8.1 | 53 | Q9Z247.1 | 84 | Q9SCY3.1 | 115 | Q10175.1 |
| 23 | Q92Q12.1 | 54 | F7DF47.4 | 85 | P44760.1 | 116 | P38911.2 |
| 24 | P0CAX0.1 | 55 | K7ELI6.1 | 86 | P57599.1 | 117 | O74191.1 |
| 25 | O68129.1 | 56 | Q9VGK3.1 | 87 | Q4D932.1 | 118 | P54397.2 |
| 26 | Q9ZCB7.1 | 57 | P45878.1 | 88 | P51752.2 | 119 | Q4J171.1 |
| 27 | Q8Y7L0.1 | 58 | A0A0K0JTK3.1 | 89 | Q9JYI8.1 | 120 | Q5T1M5.2 |
| 28 | P56420.1 | 59 | Q20107.1 | 90 | Q9HVL2.1 | 121 | O22870.2 |
| 29 | Q8R5Z2.1 | 60 | Q9V3V2.1 | 91 | F5HDS0.1 | | |
| 30 | Q9K8F3.1 | 61 | P20080.1 | 92 | Q9KP11.1 | | |
| 31 | Q46108.2 | 62 | Oδ0046.1 | 93 | P0A9L3.2 | | |

**[Table 3]**

| | |
|---|---|
| 1 | P26623.3 |
| 2 | P0A0W2.1 |
| 3 | P44760.1 |
| 4 | P57599.1 |
| 5 | Q4D932.1 |
| 6 | P51752.2 |
| 7 | Q9HVL2.1 |
| 8 | F5HDS0.1 |
| 9 | Q9KP11.1 |
| 10 | P0A9L3.2 |
| 11 | Q9HYX8.1 |
| 12 | Q9A2C9.1 |
| 13 | Q9A4N1.1 |
| 14 | A0KGZ9.1 |
| 15 | O83834.1 |
| 16 | Q9KLT0.1 |
| 17 | Q8PAI1.1 |
| 18 | Q9KV04.1 |
| 19 | Q8XZ41.1 |
| 20 | Q9RJ63.1 |
| 21 | P30417.2 |
| 22 | FkpA |

### (2) Identification of FKBP sequence group belonging to BCFK clade

The FKBP sequence group belonging to the BCFK clade, which is shown in Table 3, was summarized in the Fasta format to provide a file BCFK.fasta. The command of mafft --auto BCFK_mafft.fasta>BCFK.fasta was executed in MAFFT v7.471 to construct multiple alignment. Next, the command of hmmbuild --pnone --wnone BCFK_mafft.hmm BCFK_mafft.fasta was executed using HMMER version 3.3.2 to prepare an HMM profile illustrated in Fig. 6, which was further binarized by the execution of the command of hmmpress BCFK_mafft.hmm. An FKBP sequence group belonging to no BCFK clade in the FKBP sequence group included in Table 2 was summarized in the Fasta format to provide a file No_BCFK.Fasta. The command of hmmscan -- tblout BCFK.tblout BCFK_mafft.hmm No_BCFK.Fasta was executed to perform the homology search of the HMM profile which defined the BCFK clade for sequences excluded from the BCFK clade among the FKBP sequences. As a result, all the sequences excluded from the BCFK clade were confirmed to have an HMM score of 112.3 or less. Next, PPIase (PrsA, FkpA, FKBP_l, FKBP_K, FKBP_E, FKBP_P, MJFKS, SurA, PpiA, Tig, FKBP2, FK153, FKBP52, and CyP40) sequences of SEQ ID NOs: 18 and 81 to 93 were summarized in the Fasta format to provide a file Express_PPIase.Fasta. The command of hmmscan --tblout BCFK.tblout BCFK_mafft.hmm Express_PPIase.Fasta was executed to perform the homology search of the HMM profile which defined the BCFK clade for the PPIase sequences described in Example 1. The HMM scores of sequences which exhibited no homology were indicated as 0. The results are shown in Table 4. FkpA, FKBP_l, FKBP_K, FKBP_E, and FKBP_P which exhibited a high productivity-improving effect in 1-6 of Example 1 had an HMM score of 150 or more and can thus be regarded as FKBP sequences belonging to the BCFK clade. In this way, whether an arbitrary PPIase sequence is a PPIase sequence belonging to the BCFK clade can be confirmed.

**[Table 4]**

| Sequence name | SEQ ID NO | HMM score |
|---|---|---|
| PrsA | 18 | 0 |
| SurA | 87 | 0 |
| PpiA | 88 | 0 |
| Tig | 89 | 0 |
| FkpA | 81 | 442 |
| FKBP_I | 82 | 401.7 |
| FKBP_K | 83 | 366 |
| FKBP_E | 84 | 308.9 |
| FKBP_P | 85 | 150.2 |
| FKBP2 | 90 | 64.9 |
| FK153 | 91 | 9.9 |
| MJFKS | 86 | 0 |
| FKBP52 | 92 | 26 |
| CyP40 | 93 | 0 |

## Claims

1. A method for producing a protein of interest, comprising: culturing a gram-positive bacterium comprising a gene encoding heterologous FK506-binding protein (FKBP) and a gene encoding the protein of interest; or culturing a mixture of a gram-positive bacterium comprising the gene encoding heterologous FKBP and a gram-positive bacterium comprising the gene encoding the protein of interest.

2. The method according to claim 1, wherein the heterologous FKBP is a polypeptide which belongs to a BCFK clade and has an isomerization activity of a prolyl bond, a polypeptide consisting of an amino acid sequence of SEQ ID NO: 6, or a polypeptide which consists of an amino acid sequence having at least 80% identity to the amino acid sequence of SEQ ID NO: 6 and has an isomerization activity of a prolyl bond.

3. The method according to claim 1, wherein the heterologous FKBP is a polypeptide consisting of any of amino acid sequences of SEQ ID NOs: 1 to 6, or a polypeptide which consists of an amino acid sequence having at least 80% identity to any of the amino acid sequences of SEQ ID NOs: 1 to 6 and has an isomerization activity of a prolyl bond.

4. The method according to any one of claims 1 to 3, wherein the gene encoding heterologous FKBP is operably linked to a polynucleotide encoding a secretory signal peptide.

5. The method according to any one of claims 1 to 4, wherein the gram-positive bacterium is *Bacillus subtilis* or a mutant strain thereof.

6. The method according to any one of claims 1 to 4, wherein the gram-positive bacterium is *Bacillus subtilis* with at least one of extracellular protease gene deleted or inactivated.

7. The method according to any one of claims 1 to 6, wherein the protein of interest has at least one prolyl bond isomerization site.

8. The method according to any one of claims 1 to 7, wherein the protein of interest is an antibody-related molecule.

9. The method according to any one of claims 1 to 7, wherein the protein of interest is a Fab.

10. A gram-positive bacterium comprising a gene encoding heterologous FKBP, or the gene encoding heterologous FKBP and a gene encoding a protein of interest.
